# EUROPEAN PATENT SPECIFICATION

(11) **EP 3 610 055 B1**
(45) Date of publication and mention of the grant of the patent: **24.05.2023**
(21) Application number: 18724015.5
(22) Date of filing: 11.04.2018
(51) Int. Cl.: D01F 1/10, D01F 6/70, B32B 5/04

(54) **ELASTIC FIBER WITH REDUCED SURFACE FRICTION AND TACK**
ELASTISCHE FASER MIT REDUZIERTER OBERFLÄCHENREIBUNG UND REDUZIERTER KLEBRIGKEIT
FIBRE ÉLASTIQUE À FRICTION DE SURFACE RÉDUITE ET COLLANT RÉDUIT

(30) Priority: 12.04.2017 US 201762484634 P
(43) Date of publication of application: 19.02.2020
(73) Proprietor: The LYCRA Company UK Limited, Manchester M2 3DE (GB)
(72) Inventor: BING-WO, Ronald D., Waynesboro, Virginia 22980 (US)
(74) Representative: Carpmaels & Ransford LLP
(86) International application number: PCT/US2018/027042
(87) International publication number: WO 2018/191345

(56) References cited:
- US-A1- 2004 127 128
- US-A1- 2013 298 519
- US-B1- 6 784 127

## Description

### BACKGROUND

Spandex elastomeric yarns can offer high stretch, good recovery from extension and good fit to the articles made from them, such as weft knit, warp knit, woven fabrics and other textiles. However, the spandex substrate suffers from high tack and friction as compared to conventional, inelastic fibers which can limit commercial applications. Because of their increased tackiness, spandex filaments may cohere to each other or alternatively adhere to various surfaces. High tackiness becomes especially problematic in packaging where spandex filament is wound around a core. The close proximity of the fibers plus the pressure on the fibers, especially near the core, may cause adjacent pieces of filament to adhere to each other, leaving the effected filament unusable since the fibers can be difficult to remove from the wound package without breaking. If the fiber does not break, it may still experience highly variable elongation due to tack points and associated tension spikes. Unusable filament commonly occurs at the core and is referred to as "core waste". After packaging, filament tackiness may increase during storage depending on time and temperature. Longer storage time and higher temperatures equate to increased tackiness and more core waste than freshly spun and packaged spandex. Accordingly, a reduction in spandex tackiness would reduce core waste, improve unwinding performance, and increase cost effectiveness.

Further relevant in information can be found in documents US 2013/298519, US 2004/127128 and US 6 784 127.

### SUMMARY

Briefly described, embodiments of this disclosure include an elastic fiber containing a wax incorporated into the fiber to reduce surface friction and over-end-take-off tack without negatively impacting creep performance, methods of preparing the fiber, methods of using this fiber, laminates including the fiber, fabrics including the fiber, garments, textiles including the fiber, and the like.

One exemplary elastic fiber, among others, comprises: polyurethane or polyurethaneurea or a mixture thereof and about 0.25% to about 4% by weight of a wax composition, wherein the wax composition comprises a polyolefin wax. The elastic fiber further comprises at least one additive selected from the group consisting of: calcium stearate, magnesium stearate, organic stearate, silicon oil, mineral oil, and mixtures thereof. In addition, an embodiment of the present disclosure includes a fabric comprising an elastic fiber as described herein. In addition, an embodiment of the present disclosure includes a laminate comprising an elastic fiber as described herein. In addition, an embodiment of the present disclosure includes a garment comprising an elastic fiber as described herein.

A process for preparing an elastic fiber, among others, comprises: a) preparing a composition comprising at least one polyurethane, polyurethaneurea, or a mixture thereof; and (b) adding to the composition about 0.25% to 4% by weight of a wax composition, wherein the wax composition comprises a polyolefin wax; (c) adding to the composition at least one additive selected from the group consisting of: calcium stearate, magnesium stearate, organic stearate, silicon oil, mineral oil, and mixtures thereof; and (d) preparing fiber from the composition by a spinning process selected from the group consisting of: wet spinning, dry spinning and melt spinning.

### BRIEF DESCRIPTION OF THE DRAWINGS

FIG. 1 is an interval plot of surface friction coefficient comparing elastic fibers of the present invention with control (T837 LYCRA HyFit^{®} fiber) and developmental (D59) fiber, by INVISTA.
FIG. 2 is a bar graph comparing over-end-take-off tack or tension (OETOT) measured at the package surface and near the core in elastic fibers of the present invention compared with cntrl (T837 LYCRA HyFit^{®} fiber) and developmental (D59) fiber by INVISTA.
FIG. 3 is an interval plot of yarn creep performance comparing elastic fibers of the present invention with cntrl (T837 LYCRA HyFit^{®} fiber) and developmental (D59) fiber by INVISTA.

### DETAILED DESCRIPTION

Before the present disclosure is described in greater detail, it is to be understood that this disclosure is not limited to particular embodiments described, as such may, of course, vary. It is also to be understood that the terminology used herein is for the purpose of describing particular embodiments only, and is not intended to be limiting, since the scope of the present disclosure will be limited only by the appended claims.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure, the preferred methods and materials are now described.

All publications and patents cited in this specification are herein incorporated by reference as if each individual publication or patent were specifically and individually indicated to be incorporated by reference and are incorporated herein by reference to disclose and describe the methods and/or materials in connection with which the publications are cited. The citation of any publication is for its disclosure prior to the filing date and should not be construed as an admission that the present disclosure is not entitled to antedate such publication by virtue of prior disclosure. Further, the dates of publication provided could be different from the actual publication dates that may need to be independently confirmed.

As will be apparent to those of skill in the art upon reading this disclosure, each of the individual embodiments described and illustrated herein has discrete components and features that may be readily separated from or combined with the features of any of the other several embodiments without departing from the scope or spirit of the present disclosure. Any recited method can be carried out in the order of events recited or in any other order that is logically possible.

Embodiments of the present disclosure will employ, unless otherwise indicated, techniques of chemistry, fabrics, textiles, and the like, which are within the skill of the art. Such techniques are fully explained in the literature.

The following examples are put forth so as to provide those of ordinary skill in the art with a complete disclosure and description of how to perform the methods and use the compositions and compounds disclosed and claimed herein. Efforts have been made to ensure accuracy with respect to numbers (e.g., amounts, temperature, etc.), but some errors and deviations should be accounted for. Unless indicated otherwise, parts are parts by weight, temperature is in °C, and pressure is in atmospheres. Standard temperature and pressure are defined as 25°C and 1 atmosphere.

Before the embodiments of the present disclosure are described in detail, it is to be understood that, unless otherwise indicated, the present disclosure is not limited to particular materials, reagents, reaction materials, manufacturing processes, or the like, as such can vary. It is also to be understood that the terminology used herein is for purposes of describing particular embodiments only, and is not intended to be limiting. It is also possible in the present disclosure that steps can be executed in different sequence where this is logically possible.

It must be noted that, as used in the specification and the appended claims, the singular forms "a," "an," and "the" include plural referents unless the context clearly dictates otherwise. Thus, for example, reference to "a support" includes a plurality of supports. In this specification and in the claims that follow, reference will be made to a number of terms that shall be defined to have the following meanings unless a contrary intention is apparent.

### Definitions

As used herein, the term "fiber" refers to filamentous material that can be used in fabric and yarn as well as textile fabrication. One or more fibers can be used to produce a fabric or yarn. The yarn can be fully drawn or textured according to methods known in the art.

As used herein, "spandex" refers to synthetic fiber in which the fiber-forming substance is a long chain synthetic elastomer comprised of about 85% or more by weight of a segmented polyurethane, where polyurethaneureas are considered a sub-class of such polyurethanes. Such a synthetic fiber may be wound on a cylindrical core to form a supply package. Spandex compositions may be prepared by a wet-spinning or a dry-spinning process and can have any of a variety of cross-sections such as a round cross-section or a flat "tape-like" cross section. Alternatively, a polyurethane solution can be cast and dried to form a "tape" configuration.

As used herein, the phrase "wax composition" is meant to encompass any wax containing additive which, when added to an elastic fiber, decreases surface friction and/or over-end-take-off tack or tension as compared to an elastic fiber without the wax containing additive.

### Discussion

Embodiments of the present disclosure provide for an elastic fiber containing a wax composition incorporated into the fiber, methods of preparing the fiber, methods of using this fiber, laminates including the fiber, fabrics including the fiber, garments including the fiber, textiles including the fiber, and the like. Embodiments of the present disclosure provide elastic fibers that provide good delivery of the fiber from the package or core. Embodiments of the present disclosure provide smooth and even delivery of the elastic fiber, which may reduce pinching, breakage, and/or other damage of the fiber, as opposed to other elastic fibers that cause irregular delivery of the fibers.

Embodiments of the present disclosure include elastic or spandex fibers that include a wax composition. The wax composition can include any wax that provides an anti-tack benefit and/or to decreases in surface friction to spandex fiber such that the fiber may be used without the addition of a topical finish to the fiber. In addition, unlike most anti-tack compositions, the elastic fibers' inclusion of the wax composition does not have a deleterious effect on the adhesion of the fiber or yarn, and may even provide an enhanced adhesion of the yarn to a fabric using hot melt elastic attachment adhesives. In one nonlimiting embodiment, the wax composition comprises a polyolefin wax. Nonlimiting examples include polypropylene or polyethylene waxes or other highly modified polymer waxes including C24-C36 polymers with maleic anhydride, esters of montan wax acids. Commercial examples include Ceridust 5551, Ceridust 8020 and Ceridust 6050 as produced by CLARIANT. In one nonlimiting embodiment, the wax composition further comprises an additional additive such as calcium stearate, magnesium stearate, organic stearates, silicon oil, mineral oil, and mixtures thereof. The wax composition and optionally the additive or additives are added to the polyurethane or polyurethaneurea polymer prior to spinning of the fiber.

In an embodiment, the elastic fiber of the present disclosure comprises a polyurethane or polyurethaneurea and a wax composition, as well as one or more additives. Suitable additives include, but are not limited to, organic stearates (e.g., calcium stearate or magnesium stearate), mineral oil, silicon oil, and mixtures thereof. In certain embodiments, the elastic fiber or the wax composition may include at least one additional particulate anti-tack agent in addition to the wax composition noted herein.

In one nonlimiting embodiment, the elastic fiber of the present disclosure includes, for example, about 0.25% to 4% wax composition by weight of the fiber.

In one nonlimiting embodiment, the wax composition of the present disclosure is a micronized metallocene-based polypropylene wax.

In one nonlimiting embodiment, the wax composition of the present disclosure is a micronized polyethylene wax.

In one nonlimiting embodiment, the elastic fiber of the present disclosure includes an additive. In one nonlimiting embodiment, the elastic fiber may comprise about 0.1% to 1.0%, about 0.1% to 2.0%, about 0.1% to 3.0%, about 0.1% to 4.0%, about 0.1% to 5.0%, about 0.1% to 6.0%, about 0.1% to 7.0%, about 0.1% to 8.0%, about 0.1% to 9.0%, or about 0.1% to 10.0% of an additive (e.g., a stearate, a silicon oil or a mineral oil).

Embodiments of the present disclosure include a process for preparing any one of the elastic fiber as described herein. The process comprises preparing a composition including at least a polyurethane, a polyurethaneurea, or mixtures thereof. Subsequently, the process includes adding a wax composition to the composition, wherein the wax composition comprises a polyolefin wax. Next, the process includes adding an additive to the composition (e.g., calcium stearate, magnesium stearate, organic stearate, silicon oil, mineral oil, or mixtures thereof). Next, the process includes preparing a fiber from the composition by a spinning process (e.g., wet spinning, dry spinning, and melt spinning).

In one nonlimiting embodiment, the spinning process for production of the fibers is dry-spinning. Dry-spinning refers to the process of forcing a polymer solution through spinneret orifices into a shaft to form a filament. Heated inert gas is passed through the chamber, evaporating the solvent from the filament as the filament passes through the shaft. The resulting elastic fiber may then be wound on a cylindrical core to form a spandex supply package. A wet-spinning process may also be used as well as the casting and drying of the polymer solution.

In one nonlimiting embodiment, the process comprises preparing a composition containing at least one polyurethane or polyurethaneurea, or mixtures thereof, adding an additive to the composition selected from calcium stearate, magnesium stearate, organic stearate, silicon oil, mineral oil, and mixtures thereof, adding about 0.25% to 4% of wax composition to the composition, and preparing fiber from the resulting composition by a spinning process selected from wet spinning, dry spinning, and melt spinning.

In one embodiment, the polymers used to create the elastic fibers of the present disclosure are prepared by capping a macromolecular glycol with, for example, a diisocyanate, then dissolving the resulting capped glycol in a suitable solvent (e.g., dimethylacetamide (DMAc), N-methylpyrrolidone, dimethylformamide, and the like), and chain-extending the capped glycol with chain extenders such as diols to form polyurethanes, or diamines to form polyurethaneureas.

Polyurethaneurea compositions useful for preparing fiber or long chain synthetic polymers that include at least 85% by weight of a segmented polyurethane. Typically, these include a polymeric glycol which is reacted with a diisocyanate to form an NCO-terminated prepolymer (a "capped glycol"), which is then dissolved in a suitable solvent, such as dimethylacetamide, dimethylformamide, or N-methylpyrrolidone, and secondarily reacted with a difunctional chain extender.

Polyurethanes are formed in a second step when the chain extenders are diols (and may be prepared without solvent). Polyurethaneureas, a sub-class of polyurethanes, are formed when the chain extenders are diamines. In the preparation of a polyurethaneurea polymer which can be spun into spandex, the glycols are extended by sequential reaction of the hydroxy end groups with diisocyanates and one or more diamines. In each case, the glycols must undergo chain extension to provide a polymer with the necessary properties, including viscosity. If desired, dibutyltin dilaurate, stannous octoate, mineral acids, tertiary amines such as triethylamine, N,N'-dimethylpiperazine, and the like, and other known catalysts can be used to assist in the capping step.

Examples of suitable polymeric glycol components include, but are not limited to, polyether glycols, polycarbonate glycols, and polyester glycols of number average molecular weight of about 600 to 3,500. Mixtures of two or more polymeric glycol or copolymers can be included.

Examples of polyether glycols that can be used include, but are not limited to, those glycols with two hydroxyl groups, from ring-opening polymerization and/or copolymerization of ethylene oxide, propylene oxide, trimethylene oxide, tetrahydrofuran, and 3-methyltetrahydrofuran, or from condensation polymerization of a polyhydric alcohol, such as a diol or diol mixtures, with less than 12 carbon atoms in each molecule, such as ethylene glycol, 1,3-propanediol, 1,4-butanediol, 1,5-pentanediol 1,6-hexanediol, 2,2-dimethyl-1,3 propanediol, 3-methyl-1,5-pentanediol, 1,7-heptanediol, 1,8-octanediol, 1,9-nonanediol, 1,10-decanediol and 1,12-dodecanediol. A poly(tetramethylene ether) glycol of molecular weight of about 1,700 to about 2,100, such as Terathane^{®} 1800 (INVISTA of Wichita, KS) with a functionality of 2, is an example of a specific suitable glycol. Co-polymers can include poly(tetramethylene-co-ethyleneether) glycol.

Examples of polyester polyols that can be used include, but are not limited to, those ester glycols with two hydroxyl groups, produced by condensation polymerization of aliphatic polycarboxylic acids and polyols, or their mixtures, of low molecular weights with no more than 12 carbon atoms in each molecule. Examples of suitable polycarboxylic acids include, but are not limited to, malonic acid, succinic acid, glutaric acid, adipic acid, pimelic acid, suberic acid, azelaic acid, sebacic acid, undecanedicarboxylic acid, and dodecanedicarboxylic acid. Examples of suitable polyols for preparing the polyester polyols include, but are not limited to, ethylene glycol, 1,3-propanediol, 1,4-butanediol, 1,5-pentanediol 1,6-hexanediol, neopentyl glycol, 3-methyl-1,5-pentanediol, 1,7-heptanediol, 1,8-octanediol, 1,9-nonanediol, 1,10-decanediol and 1,12-dodecanediol. A linear bifunctional polyester polyol with a melting temperature of about 5°C to 50°C is an example of a specific polyester polyol.

Examples of polycarbonate polyols that can be used include, but are not limited to, those carbonate glycols with two or more hydroxy groups, produced by condensation polymerization of phosgene, chloroformic acid ester, dialkyl carbonate or diallyl carbonate and aliphatic polyols, or their mixtures, of low molecular weights with no more than 12 carbon atoms in each molecule. Examples of suitable polyols for preparing the polycarbonate polyols include, but are not limited to, diethylene glycol, 1,3-propanediol, 1,4-butanediol, 1,5-pentanediol, 1,6-hexanediol, neopentyl glycol, 3-methyl-1,5-pentanediol, 1,7-heptanediol, 1,8-octanediol, 1,9-nonanediol, 1,10-decanediol and 1,12-dodecanediol. A linear, bifunctional polycarbonate polyol with a melting temperature of about 5°C to about 50°C is an example of a specific polycarbonate polyol.

In one nonlimiting embodiment, the diisocyanate component also includes a single diisocyanate or a mixture of different diisocyanates including an isomer mixture of diphenylmethane diisocyanate (MDI) containing 4,4'-methylene bis(phenyl isocyanate) and 2,4'-methylene bis(phenyl isocyanate). Any suitable aromatic or aliphatic diisocyanate can be included. Examples of diisocyanates that can be used include, but are not limited to, 4,4'-methylene bis(phenyl isocyanate), 2,4'- methylene bis(phenyl isocyanate), 4,4'-methylenebis(cyclohexyl isocyanate), 1,3-diisocyanato-4-methyl-benzene, 2,2'-toluenediisocyanate, 2,4'-toluenediisocyanate, and mixtures thereof.

Chain extenders which may be used in some nonlimiting embodiments can be either water or a diamine chain extender for a polyurethaneurea. Combinations of different chain extenders may be included depending on the desired properties of the polyurethaneurea and the resulting fiber. Examples of suitable diamine chain extenders include, but are not limited to: hydrazine; 1,2-ethylenediamine; 1,4-butanediamine; 1,2-butanediamine; 1,3-butanediamine; 1,3-diamino-2,2-dimethylbutane; 1,6-hexamethylenediamine; 1,12-dodecanediamine; 1,2-propanediamine; 1,3-propanediamine; 2-methyl-1,5-pentanediamine; 1-amino-3,3,5-trimethyl-5-aminomethylcyclohexane; 2,4-diamino-1-methylcyclohexane; N-methylamino-bis(3-propylamine); 1,2-cyclohexanediamine; 1,4-cyclohexanediamine; 4,4'-methylene-bis(cyclohexylamine); isophorone diamine; 2,2-dimethyl-1,3-propanediamine; meta-tetramethylxylenediamine; 1,3-diamino-4-methylcyclohexane; 1,3-cyclohexane-diamine; 1,1-methylene-bis(4,4'-diaminohexane); 3-aminomethyl-3,5,5-trimethylcyclohexane; 1,3-pentanediamine (1,3-diaminopentane); m-xylylene diamine; and Jeffamine^{®} (Texaco).

When a polyurethane is desired, the chain extender is a diol. Examples of such diols that may be used include, but are not limited to, ethylene glycol, 1,3-propanediol, 1,2-propylene glycol, 3-methyl-1,5-pentanediol, 2,2-dimethyl-1,3-propanediol, 2,2,4-trimethyl-1,5-pentanediol, 2-methyl-2-ethyl-1,3-propanediol, 1,4-bis(hydroxyethoxy)benzene, and 1,4-butanediol, hexanediol and mixtures thereof.

In one nonlimiting embodiment, a monofunctional alcohol or a primary/secondary monofunctional amine may optionally be included to control the molecular weight of the polymer. Blends of one or more monofunctional alcohols with one or more monofunctional amines may also be included. Examples of monofunctional alcohols useful with the present disclosure include, but are not limited to, at least one member selected from the group consisting of aliphatic and cycloaliphatic primary and secondary alcohols with 1 to 18 carbons, phenol, substituted phenols, ethoxylated alkyl phenols and ethoxylated fatty alcohols with molecular weight less than about 750, including molecular weight less than 500, hydroxyamines, hydroxymethyl and hydroxyethyl substituted tertiary amines, hydroxymethyl and hydroxyethyl substituted heterocyclic compounds, and combinations thereof, including furfuryl alcohol, tetrahydrofurfuryl alcohol, N-(2-hydroxyethyl)succinimide, 4-(2-hydroxyethyl)morpholine, methanol, ethanol, butanol, neopentyl alcohol, hexanol, cyclohexanol, cyclohexanemethanol, benzyl alcohol, octanol, octadecanol, N,N-diethylhydroxylamine, 2-(diethylamino)ethanol, 2-dimethylaminoethanol, and 4-piperidineethanol, and combinations thereof. Examples of suitable mono-functional dialkylamine blocking agents include, but not limited to: N,N-diethylamine, N-ethyl-N-propylamine, N,N-diisopropylamine, N-tert-butyl-N-methylamine, N-tert-butyl-N-benzylamine, N,N-dicyclohexylamine, N-ethyl-N-isopropylamine, N-tert-butyl-N-isopropylamine, N-isopropyl-N-cyclohexylamine, N-ethyl-N-cyclohexylamine, N,N-diethanolamine, and 2,2,6,6-tetramethylpiperidine.

In one nonlimiting embodiment, after synthesizing the polymer solution of the present disclosure, an anti-tack additive is incorporated into the solution. The solution having the anti-tack additive dispersed therein may be dry-spun to form the elastic fiber of the present disclosure. Dry-spinning refers to the process of forcing a polymer solution through spinneret orifices into a shaft to form a filament. Heated inert gas is passed through the chamber, evaporating the solvent from the filament as the filament passes through the shaft. The resulting elastic fiber may then be wound on a cylindrical core to form a spandex supply package. A wet-spinning process may also be used as well as the casting and drying of the polymer solution.

In one nonlimiting embodiment, the elastic fiber of the present disclosure may further comprise an additional, conventional additive or additives that are added for specific purposes. Examples include, but are in no way limited to, antioxidants, thermal stabilizers, UV stabilizers, pigments and delusterants (for example titanium dioxide), dyes and dye enhancers, lubricating agents (for example silicone oil), additives to enhance resistance to chlorine degradation (for example zinc oxide; magnesium oxide and mixtures of huntite and hydromagnesite), and the like, so long as such additives do not produce antagonistic effects with the spandex elastomer or wax composition of this disclosure. Some of the conventional additives, such as titanium dioxide, exhibit small effects on over-end take-off tension (OETOT) measurements, the parameter used to judge tackiness of the elastic fiber (as described below in the Examples), but none of them has an appreciable effect on the OETOT measurements and are not added to the spandex in amounts so as to reduce tackiness.

Embodiments of the present disclosure include articles of manufacture comprising the elastic fiber of the present disclosure. These articles of manufacture include, but are not limited to, fabrics, garments, and laminate structures.

In one nonlimiting embodiment, the present disclosure provides a fabric comprising an elastic fiber which contains polyurethane or polyurethaneurea and about 0.25% to 4% by weight of wax composition, wherein the wax composition comprises a polyolefin wax. An additional additive is included, such as calcium stearate, magnesium stearate, organic stearate, silicon oil, mineral oil, and mixtures thereof. Fabrics of the present invention include, but are not limited to, those with a knit, woven, or non-woven construction

In one nonlimiting embodiment, the laminate structure comprises an elastic fiber of the present disclosure which has a polyurethane or polyurethaneurea, about 0.25% to 4% by weight of wax composition and at least one additional additive, such as calcium stearate, magnesium stearate, organic stearate, silicon oil, mineral oil, and mixtures thereof. In certain embodiments, the fiber is adhered to one or more layers of a substrate, such as a fabric, nonwoven, film, and combinations thereof. The laminate structure may be adhered by an adhesive, ultrasonic bonding, thermal bonding, mechanical bonding, or combinations thereof. The laminate structure formed with the elastic fiber of the present invention may be used in disposable hygiene articles such as, but not limited to, diapers, training pants, adult incontinence articles, or feminine hygiene articles.

### EXAMPLES

Having described the embodiments of the present disclosure, in general, the following Examples describe some additional embodiments of the present disclosure. While embodiments of present disclosure are described in connection with the following examples and the corresponding text and figures, there is no intent to limit embodiments of the present disclosure to this description. On the contrary, the intent is to cover all alternatives, modifications, and equivalents included within the spirit and scope of embodiments of the present disclosure.

### Example 1

Surface friction coefficient was determined as described in U.S. Patent No. 9,487,889, col. 11, lines 57-68 and col. 12, lines 1-14, which is incorporated herein by reference. Measurement is made collecting readings every 5 cm over 100 m length using a speed of 46 mlmin and 2.78X draft from first to last roll.

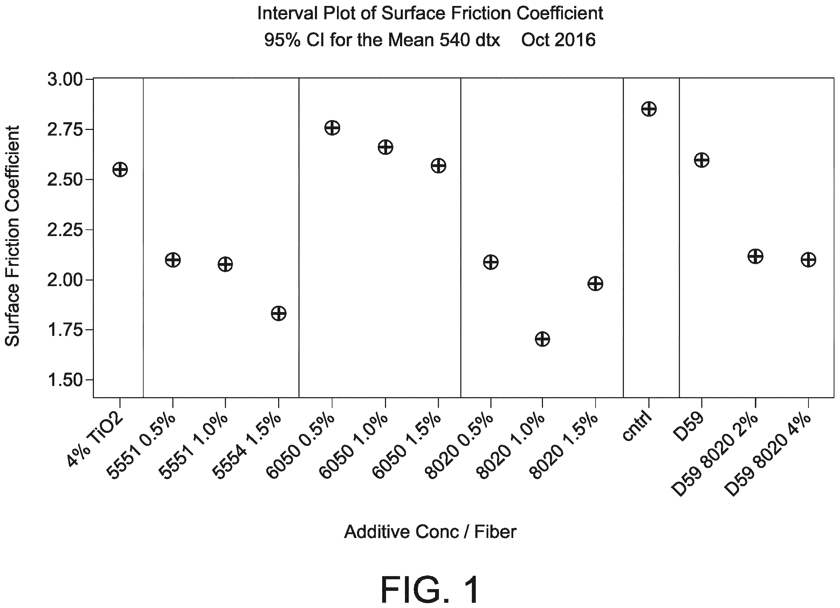

An elastic fiber with a wax composition was prepared according to the following synthetic methods. Ceridust 5551, Ceridust 8020 and Ceridust 6050 was spun into about 486 denier (540 decitex) polyurethaneurea fiber without finish on a dry spin machine. The wax composition significantly reduced yarn surface friction coefficient as noted in Figure 1.

The polymer used in this example was made by capping a 1800 molecular weight polytetramethyleneether glycol with MDI at a molar ratio of 1.69. The resulting capped glycol was chain extended in DMAc solvent with a mixture of ethylene diamine and 2-methyl-1,5-pentanediamine ((90/10 mole ratio) and terminated with diethyl amine. In addition to the wax composition, the polymer also contained: 1.4 wt.% Irgonox antioxidant available from Ciba, 0.5 wt.% Methacrol 2462B UV stabilizer available from E. I. du Pont de Nemours, Inc. 3.9% Ultracarb, a huntitte/hydromagnesite mineral mixture available from Microfine Minerals, Ltd., 0.42 wt.% silicon oil composed of 96% polydimethylsiloxane and 4 wt.% polydiamylsiloxane, and 0.3 wt.% titanium dioxide delustrant, available from E. I. du Pont de Nemours, Inc.

### Example 2

Over-end take-off tension (OETOT) was determined as described in U.S. Patent No. 4,296,174, col. 4, lines 20-45, and FIG 6, which is incorporated herein by reference. Measurement is made of the average tensile load required to remove a 183 m length of sample of spandex yarn from a tubular supply package of the yarn at a delivery rate of 45.7 m/min. In the example below, measurements were made at the surface and core of the package. For example, measurements are made after a few grams of fiber are removed to establish the intended winding pattern, i.e. "surface"; and measurements are made after all but roughly 100 g of the fiber has been removed from the package, i.e. "core".

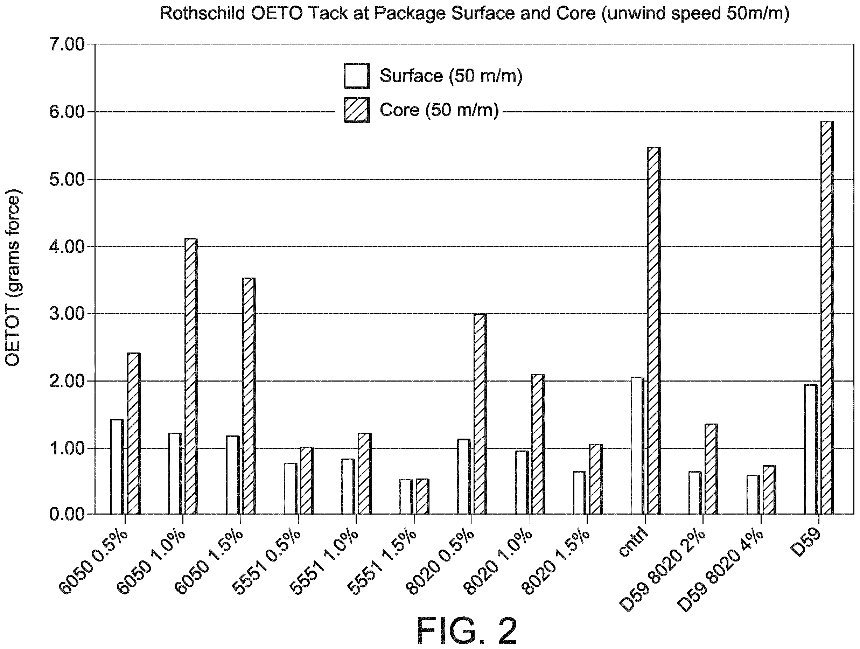

An elastic fiber with a wax composition was prepared according to the following synthetic methods. Ceridust 5551, Ceridust 8020 and Ceridust 6050 was spun into about 486 denier (540 decitex) polyurethaneurea fiber without finish on a dry spin machine. The wax composition significantly reduced yarn tackiness as quantified by OETOT in FIG. 2.

The polymer used in this example was made by capping a 1800 molecular weight polytetramethyleneether glycol with MDI at a molar ratio of 1.69. The resulting capped glycol was chain extended in DMAc solvent with a mixture of ethylene diamine and 2-methyl-1,5-pentanediamine ((90/10 mole ratio) and terminated with diethyl amine. In addition to the wax composition, the polymer also contained: 1.4 wt.% Irgonox antioxidant available from Ciba, 0.5 wt.% Methacrol 2462B UV stabilizer available from E. I. du Pont de Nemours, Inc. 3.9% Ultracarb, a huntitte/hydromagnesite mineral mixture available from Microfine Minerals, Ltd., 0.42 wt.% silicon oil composed of 96% polydimethylsiloxane and 4 wt.% polydiamylsiloxane, and 0.3 wt.% titanium dioxide delustrant, available from E. I. du Pont de Nemours, Inc.

### Example 3

Yarn creep is determined after construction a laminate consisting of the spandex fibers that are elongated and adhered using elastic attachment adhesive between two layers of nonwoven fabric, typically polypropylene. The laminate is allowed to relax and contract. The laminate is stretched so that the nonwoven layers are smooth and then it is attached to a support frame by the ends. A predetermined length of spandex fibers within the support frame is measured and marked. The ends of the individual spandex fibers are cut at the marks. The laminate and support frame is stored for four hours at 40°C. The length of the spandex fibers is measured after storage. The percent difference in the contracted length of cut spandex and the predetermined length is reported as percent yarn creep.

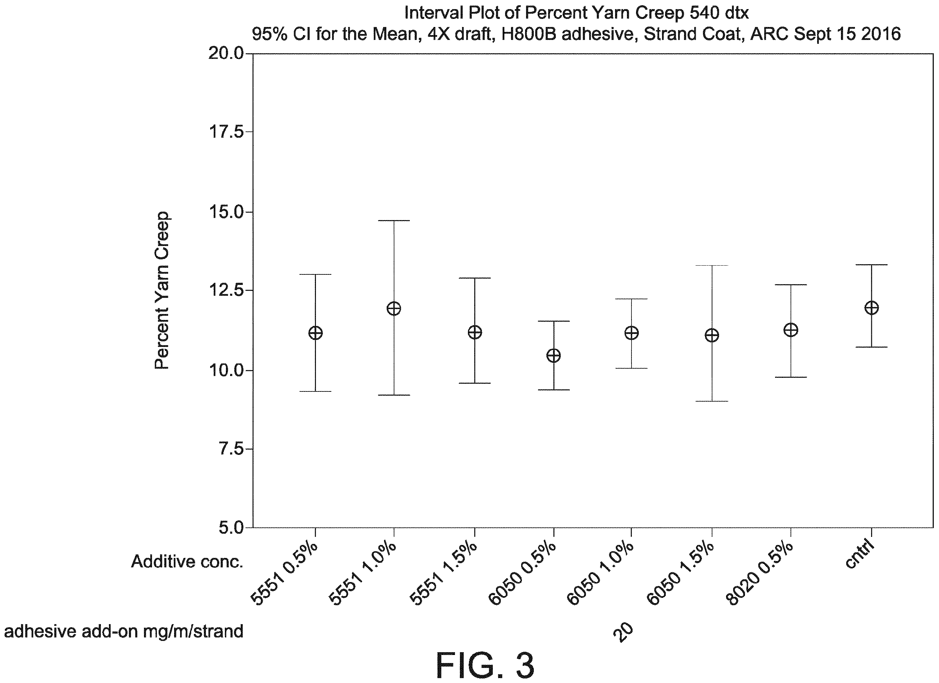

Figure 3 indicates the addition of wax to the fiber does not degrade the adhesion of the spandex fibers as the percent yarn creep is very similar between all the samples and control fiber.

It should be noted that ratios, concentrations, amounts, and other numerical data may be expressed herein in a range format. It is to be understood that such a range format is used for convenience and brevity, and thus, should be interpreted in a flexible manner to include not only the numerical values explicitly recited as the limits of the range, but also to include all the individual numerical values or sub-ranges encompassed within that range as if each numerical value and sub-range is explicitly recited. To illustrate, a concentration range of "about 0.1% to about 5%" should be interpreted to include not only the explicitly recited concentration of about 0.1 wt% to about 5 wt%, but also the individual concentrations (e.g., 1%, 2%, 3%, and 4%) and the sub-ranges (e.g., 0.5%, 1.1%, 2.2%, 3.3%, and 4.4%) within the indicated range. The term "about" can include ±1%, ±2%, ±3%, ±4%, ±5%, ±8%, or ±10%, of the numerical value(s) being modified. In addition, the phrase "about 'x' to 'y'" includes "about 'x' to about 'y'".

## Claims

1. An elastic fiber comprising polyurethane or polyurethaneurea and about 0.5% to 4% by weight of a wax composition, wherein the wax composition comprises a polyolefin wax.

2. The elastic fiber of claim 1, further comprising at least one particulate anti-tack agent and/or a topically applied mineral oil or silicon oil, or blends containing mineral oil or silicon oil as a spin finish in an amount of about 0.5% to 2.0% by weight of the final fiber once applied.

3. The elastic fiber of claim 1, wherein said fiber excludes a spin finish.

4. A process for preparing an elastic fiber comprising:
(a) preparing a composition including at least one polyurethane, polyurethaneurea, or a mixture thereof;
(b) adding to the composition about 0.25% to 4% by weight of a wax composition, wherein the wax composition comprises a polyolefin wax;
(c) adding to the composition at least one additive selected from the group consisting of: calcium stearate, magnesium stearate, organic stearate, silicon oil, mineral oil, and mixtures thereof; and
(d) preparing fiber from the composition by a spinning process selected from the group consisting of wet spinning, dry spinning and melt spinning.

5. A fabric comprising an elastic fiber comprising polyurethane or polyurethaneurea and about 0.25% to 4% by weight of a wax composition, wherein the wax composition comprises a polyolefin wax.

6. A laminate structure comprising:
an elastic fiber comprising polyurethane or polyurethaneurea and about 0.25% to 4% by weight of a wax composition, wherein the wax composition comprises a polyolefin wax;
wherein said fiber is adhered to one or more layers of a substrate selected from the group consisting of fabric, nonwoven, film, and combinations thereof.

7. The elastic fiber of claim 1, the process of claim 4, the fabric of claim 5 or the laminate of claim 6, wherein the polyolefin wax is a micronized metallocene-based polypropylene wax.

8. The elastic fiber of claim 1, the process of claim 4 or the laminate of claim 6, wherein the wax composition comprises a modified polymer wax including C24-C36 polymers with maleic anhydride or wherein the wax composition comprises esters of montan wax acids.

9. The elastic fiber of claim 1, the process of claim 4, the fabric of claim 5 or the laminate of claim 6, wherein the polyolefin wax is a micronized polyethylene wax.

10. The elastic fiber of claim 1, the fabric of claim 5 or the laminate of any of claims 6-9, wherein the elastic fiber further comprises at least one additional additive selected from the group consisting of calcium stearate, magnesium stearate, organic stearates, silicon oil, mineral oil, and mixtures thereof.

11. The fabric of any one of claims 5, 7, 9 and 10 wherein said fabric comprises a knit, woven, or non-woven construction.

12. A garment comprising the fabric of any one of claims 5, 7, 9 and 10.

13. The laminate of any one of claims 6-9, wherein said laminate is adhered by an adhesive, ultrasonic bonding, thermal bonding, mechanical bonding, or combinations thereof.

14. A disposable hygiene article comprising the laminate of any one of claims 6-10 or 13, comprising a disposable hygiene article.

15. The disposable hygiene article of claim 14 selected from the group consisting of diapers, training pants, adult incontinence articles and feminine hygiene articles.

## Patentansprüche

1. Elastische Faser, umfassend Polyurethan oder Polyurethan-Harnstoff und etwa 0,5 Gew.% bis 4 Gew.% einer Wachszusammensetzung, wobei die Wachszusammensetzung ein Polyolefinwachs umfasst.

2. Elastische Faser nach Anspruch 1, des Weiteren umfassend mindestens ein partikuläres klebungsverhinderndes Mittel und/oder ein topisch aufgebrachtes Mineralöl oder Silikonöl oder Gemische, die Mineralöl oder Silikonöl enthalten, als Spinnzusatz in einer Menge von etwa 0,5 Gew.% bis 2,0 Gew.% der fertigen Faser nach der Auftragung.

3. Elastische Faser nach Anspruch 1, wobei die Faser einen Spinnzusatz ausschließt.

4. Verfahren zur Herstellung einer elastischen Faser, umfassend:
(a) Herstellen einer Zusammensetzung, die mindestens ein Polyurethan, einen Polyurethan-Harnstoff oder eine Mischung davon einschließt;
(b) Zufügen von etwa 0,25 Gew.% bis 4 Gew.% einer Wachszusammensetzung, wobei die Wachszusammensetzung ein Polyolefinwachs umfasst, zu der Zusammensetzung;
(c) Zufügen mindestens eines Additivs ausgewählt aus der Gruppe bestehend aus Calciumstearat, Magnesiumstearat, organischem Stearat, Silikonöl, Mineralöl und Mischungen davon zu der Zusammensetzung; und
(d) Herstellen von Fasern aus der Zusammensetzung mittels eines Spinnverfahrens ausgewählt aus der Gruppe bestehend aus Nassspinnen, Trockenspinnen und Schmelzspinnen.

5. Textilmaterial, umfassend eine elastische Faser, umfassend Polyurethan oder Polyurethan-Harnstoff und etwa 0,25 Gew.% bis 4 Gew.% einer Wachszusammensetzung, wobei die Wachszusammensetzung ein Polyolefinwachs umfasst.

6. Laminatstruktur, umfassend:
eine elastische Faser, umfassend Polyurethan oder Polyurethan-Harnstoff und etwa 0,25 Gew.% bis 4 Gew.% einer Wachszusammensetzung, wobei die Wachszusammensetzung ein Polyolefinwachs umfasst;
wobei die Faser an eine oder mehrere Schichten eines Substrats ausgewählt aus der Gruppe bestehend aus Textilmaterial, Vlies, Folie und Kombinationen davon angehaftet wird.

7. Elastische Faser nach Anspruch 1, Verfahren nach Anspruch 4, Textilmaterial nach Anspruch 5 oder Laminat nach Anspruch 6, wobei das Polyolefinwachs ein mikronisiertes metallocenbasiertes Polypropylenwachs ist.

8. Elastische Faser nach Anspruch 1, Verfahren nach Anspruch 4 oder Laminat nach Anspruch 6, wobei die Wachszusammensetzung ein modifiziertes Polymerwachs umfasst, das C₂₄- bis C₃₆-Polymere mit Maleinsäureanhydrid einschließt, oder wobei die Wachszusammensetzung Ester von Montanwachssäuren umfasst.

9. Elastische Faser nach Anspruch 1, Verfahren nach Anspruch 4, Textilmaterial nach Anspruch 5 oder Laminat nach Anspruch 6, wobei das Polyolefinwachs ein mikronisiertes Polyethylenwachs ist.

10. Elastische Faser nach Anspruch 1, Textilmaterial nach Anspruch 5 oder Laminat nach einem der Ansprüche 6 bis 9, wobei die elastische Faser des Weiteren mindestens ein zusätzliches Additiv ausgewählt aus der Gruppe bestehend aus Calciumstearat, Magnesiumstearat, organischen Stearaten, Silikonöl, Mineralöl und Mischungen davon umfasst.

11. Textilmaterial nach einem der Ansprüche 5, 7, 9 und 10, wobei das Textilmaterial eine gestrickte, gewebte oder Vlieskonstruktion umfasst.

12. Kleidungsstück, umfassend das Textilmaterial nach einem der Ansprüche 5, 7, 9 und 10.

13. Laminat nach einem der Ansprüche 6 bis 9, wobei das Laminat durch einen Klebstoff, Ultraschallbonden, thermisches Bonden, mechanisches Bonden oder Kombinationen angehaftet wird.

14. Einweg-Hygieneartikel, umfassend das Laminat nach einem der Ansprüche 6 bis 10 oder 13, umfassend einen Einweg-Hygieneartikel.

15. Einweg-Hygieneartikel nach Anspruch 14 ausgewählt aus der Gruppe bestehend aus Windeln, Trainingshosen für die Sauberkeitserziehung, Inkontinenzartikeln für Erwachsene sowie Damenhygieneartikeln.

## Revendications

1. Fibre élastique comprenant du polyuréthane ou du polyuréthane-urée et environ 0,5 % à 4 % en poids d'une composition de cire, dans laquelle la composition de cire comprend une cire de polyoléfine.

2. Fibre élastique selon la revendication 1, comprenant en outre au moins un agent anti-collant particulaire et/ou une huile minérale ou une huile silicone appliquée de façon topique, ou des mélanges contenant une huile minérale ou une huile silicone en tant qu'ensimage de filature en une quantité d'environ 0,5 % à 2,0 % en poids de la fibre finale une fois appliquée.

3. Fibre élastique selon la revendication 1, ladite fibre excluant un ensimage de filature.

4. Procédé de préparation d'une fibre élastique comprenant :
(a) la préparation d'une composition comprenant au moins un polyuréthane, un polyuréthane-urée ou un mélange de ceux-ci ;
(b) l'ajout à la composition d'environ 0,25 % à 4 % en poids d'une composition de cire, la composition de cire comprenant une cire de polyoléfine ;
(c) l'ajout à la composition d'au moins un additif choisi dans le groupe constitué de : stéarate de calcium, stéarate de magnésium, stéarate organique, huile silicone, huile minérale, et des mélanges de ceux-ci ; et
(d) la préparation d'une fibre à partir de la composition par un procédé de filature choisi dans le groupe constitué d'une filature au mouillé, une filature au sec et une filature à l'état fondu.

5. Tissu comprenant une fibre élastique comprenant du polyuréthane ou du polyuréthane-urée et environ 0,25 % à 4 % en poids d'une composition de cire, la composition de cire comprenant une cire de polyoléfine.

6. Structure de stratifié comprenant :
une fibre élastique comprenant du polyuréthane ou du polyuréthane-urée et environ 0,25 % à 4 % en poids d'une composition de cire, dans laquelle la composition de cire comprend une cire de polyoléfine ;
dans laquelle ladite fibre est collée à une ou plusieurs couches d'un substrat choisi dans le groupe constitué d'un tissu, un non-tissé, un film, et des combinaisons de ceux-ci.

7. Fibre élastique selon la revendication 1, procédé selon la revendication 4, tissu selon la revendication 5 ou stratifié selon la revendication 6, dans lesquels la cire de polyoléfine est une cire de polypropylène à base de métallocène micronisé.

8. Fibre élastique selon la revendication 1, procédé selon la revendication 4 ou stratifié selon la revendication 6, dans lesquels la composition de cire comprend une cire de polymère modifié comprenant des polymères en C24-C36 avec de l'anhydride maléique ou dans lesquels la composition de cire comprend des esters d'acides de cire de Montan.

9. Fibre élastique selon la revendication 1, procédé selon la revendication 4, tissu selon la revendication 5 ou le stratifié selon la revendication 6, dans lesquels la cire de polyoléfine est une cire de polyéthylène micronisée.

10. Fibre élastique selon la revendication 1, tissu selon la revendication 5 ou stratifié selon l'une quelconque des revendications 6 à 9, dans lesquels la fibre élastique comprend en outre au moins un additif supplémentaire choisi dans le groupe constitué de : stéarate de calcium, stéarate de magnésium, stéarates organiques, huile silicone, huile minérale, et des mélanges de ceux-ci.

11. Tissu selon l'une quelconque des revendications 5, 7, 9 et 10, ledit tissu comprenant une construction tricotée, tissée ou non tissée.

12. Vêtement comprenant le tissu selon l'une quelconque des revendications 5, 7, 9 et 10.

13. Stratifié selon l'une quelconque des revendications 6 à 9, ledit stratifié étant collé par un adhésif, collage par ultrasons, collage thermique, collage mécanique, ou des combinaisons de ceux-ci.

14. Article d'hygiène jetable comprenant le stratifié selon l'une quelconque des revendications 6 à 10 ou 13, comprenant un article d'hygiène jetable.

15. Article d'hygiène jetable selon la revendication 14 choisi dans le groupe constitué de couches, culottes de propreté, articles pour l'incontinence des adultes et articles d'hygiène féminine.
